# EUROPEAN PATENT APPLICATION

(11) **EP 0 538 510 A1**
(43) Date of publication of application: **28.04.1993**
(21) Application number: 91118079.2
(22) Date of filing: 23.10.1991
(51) Int. Cl.: A61N 1/34

(54) **Apparatus for low energy emission therapy**

(71) Applicant: SYMTONIC S.A., CH-1020 Renens (CH)
(72) Inventor: Kuster, Niels, Prof. Dr., CH-8005 Zürich (CH)
(74) Representative: Spott, Gottfried, Dr.

(57) **Abstract**

The invention concerns an apparatus for low energy emission therapy of a patient suffering from neurovegetative disorders, such as anxiety neurosis, sleep disorders including psychophysiologic insomnia, mild psychiatric disorders or other disorders of the Central Nervous System. The apparatus comprises a high frequency generator circuit for generating a high frequency low energy carrier signal having spectral frequency components within defined limits, and a modulation signal for modulating the amplitude of the carrier signal with a frequency of modulation within defined limits, a power source for activating the generator means, an emitter (10) for emitting the modulated signal, an antenna cable (12) connected at its proximal end to the emitter and connected at its distal end to a probe connection device for connection to the patient to be treated, and an impedance transformer (14) provided between the emitter (10) and the probe connection device to match the impedance of the patient seen from the emitter (10) with that of the output circuit of the high frequency generator circuit.

## Description

This invention relates to an apparatus for low energy emission therapy (known as a LEET apparatus or LEET device).

### BACKGROUND OF THE INVENTION

Low Energy Emission Therapy involving emissions of low energy electromagnetic waves of radio type has been found to be an effective mode of treating a patient suffering from neurovegetative disorders such as anxiety neurosis, sleep disorders including psychophysiologic insomnia, mild psychiatric disorders or other disorders of the Central Nervous System. Apparatus for carrying out such treatment is described in our earlier patents, e.g. including USP 4,649,935 and USP 4,765,322. At the time of these earlier disclosures, little was understood about the mechanisms of the treatment or how to secure best results. More particularly it was not appreciated how to achieve transmission of electromagnetic fields or transfer of power to the patient and what factors need to be considered. Also, by virtue of the electromatnetic fields transmitted being variable dependently of such parameters as patient movement or position, it was not possible to achieve controlled transmissions to the patient and hence difficult to achieve controlled results of treatment. It is an object of this invention to substantially eliminate the effects of patient movement on the transfer of electromagnetic wave energies to the patient and to obtain effective energy transfer.

### SUMMARY OF THE INVENTION

It has been found, in accordance with the invention, that in order to achieve an effective transfer of power from the emitter through the antenna cable and into the patient via the connector probe, account should be taken of the fact that, in this type of power transfer of low energy wave emissions, the body of the patient behaves as an elongation of the antenna. In order however for power to transfer effectively from the antenna cable to the patient, the impedance of the patient connected to the antenna cable via the connector probe should match the impedance of the antenna cable which in turn should match the impedance of the high frequency circuit comprising the emitter. In order to achieve this matching of impedances, there is provided, in accordance with this invention, an impedance transformer between the distal end of the antenna cable and the probe connection, which impedance transformer is selected to match the impedance of the patient seen from the emitter with that of the output circuit of the high frequency generator.

The carrier signal and modulation signal may be selected to drive the emitter with an amplitude modulated signal in which the carrier signal comprise spectral frequency components of below 1 GHz and in which the modulation signal comprises spectral frequency components between 0.1 Hz and 10 kHz.

### BRIEF DESCRIPTION OF THE DRAWINGS

Figure 1 is a circuit diagram showing an illustration of a circuit which may be employed in the apparatus of the invention; and
Figure 2 is an illustration of an antenna cable and connector probe which may be comprised in the apparatus of the invention.

### DETAILED DESCRIPTION OF THE DRAWINGS

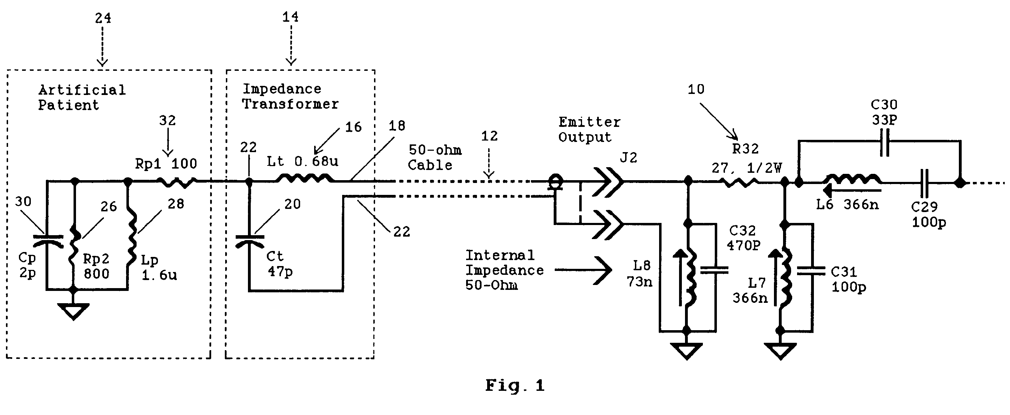

Referring to Figure 1 of the drawings, the right-hand section of the circuit shows an emitter circuit 10. The emitter is driven by a low energy carrier signal having spectral frequency components within defined limits, modulated with a frequency of modulation within defined limits. This modulated signal is generated by a generator (not shown) powered by an accumulator, preferably of rechargeable type.

The emitter circuit 10 is in the drawing indicated to possess an internal impedance of 50 ohm or a 50 ohm output impedance. This is an exemplary impedance and is chosen to match a 50 ohm coaxial antenna cable 12 impedance.

Reference numeral 14 refers generally to an impedance transformer which comprises a resistance 16 connected in the first line 18 of the coaxial cable 12 and a capacitor 20 connected in parallel in the second line 22 of the coaxial cable 12. The probe connection (not shown in Figure 1) is connected to the impedance transformer to a point 22 where the first and second lines 18 and 22 of the coaxial antenna cable 12 are connected together.

In Figure 1, for purposes of construction and testing, the probe connection and patient are replaced by what is named an artificial patient 24. The artificial patient is constructed to simulate the impedance of the average patient, which impedance is of the order of 190 + j 200 ohm. This patient impedance is approximately simulated by the resistance 26, inductor 28 and the capacitor 30 connected in parallel and by the resistance 32 connected to the impedance transformer 14. The values of the resistances and of the capacitors in the impedance transformer 14 are exemplary for 50 ohm impedances of the high frequency circuit (and coaxial cable).

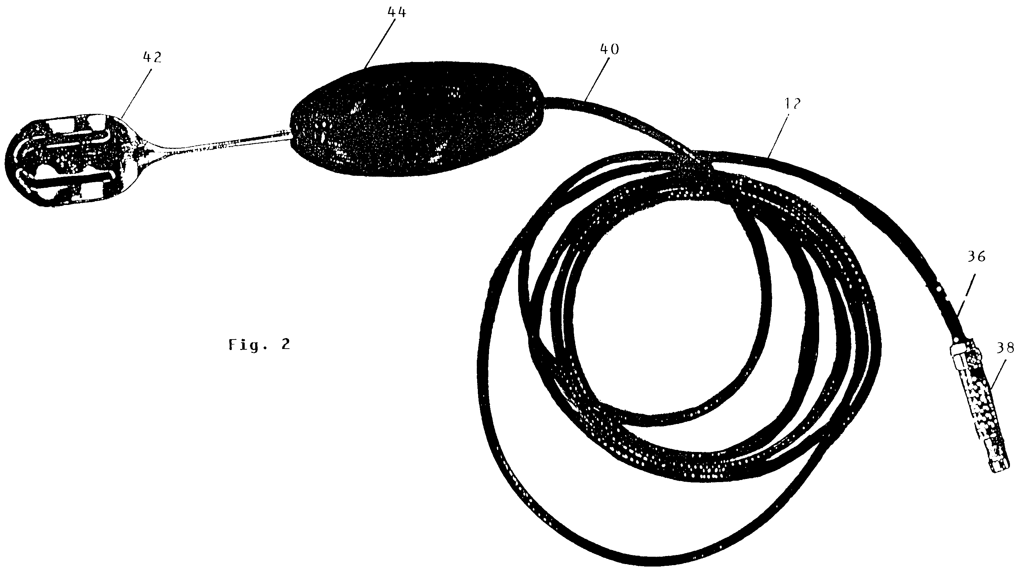

Referring to Figure 2, the coaxial antenna cable is once again referred to by the reference numeral 12. The proximal end 36 of the cable is provided with connection means 38 for coaxial connection to the emitter 10 (refer to Figure 1). The distal end 40 of the coaxial cable is connected to a probe connection device 42 which is of electrically conductive material, such as of metal, and which is shaped to fit into the mouth of a patient (not shown) and thus achieve a good electrical connection between the patient and the antenna cable. The impedance transformer 14 referred to and described in relation to Figure 1 is housed in housing 44 which is conveniently shaped to also serve as a means for holding the probe connection device.

## Claims

1. Apparatus for low energy emission therapy of a patient suffering from neurovegetative disorders, such as anxiety neurosis, sleep disorders including psychophysiologic insomnia, mild psychiatric disorders or other disorders of the Central Nervous System, comprising a high frequency generator circuit for generating a high frequency low energy carrier signal having spectral frequency components within defined limits, and a modulation signal for modulating the amplitude of the carrier signal with a frequency of modulation within defined limits, a power source for activating the generator means, an emitter for emitting the modulated signal, an antenna cable connected at its proximal end to the emitter and connected at its distal end to a probe connection device for connection to the patient to be treated, characterized in that an impedance transformer is provided between the emitter and the probe connection device to match the impedance of the patient seen from the emitter with that of the output circuit of the high frequency generator circuit.

2. Apparatus according to claim 1, in which the carrier signal and modulation signal are selected to drive the emitter with an amplitude modulated signal in which the carrier signal comprises spectral frequency components of below 1 GHz and in which the modulation signal comprises spectral frequency components between 0.1 Hz and 10 kHz.

3. Apparatus according to claim 1 or claim 2, in which the probe connection device for connection to the patient comprises a probe element of electrically conductive material shaped to fit into the mouth of the patient and thus provide efficient connection between the patient and the antenna cable leading to the emitter.

4. Apparatus according to any one of the preceding claims, in which the impedance transformer provided between the emitter and the probe connection device comprises a resistance connected in the first line of a coaxial antenna cable and a capacitor connected in parallel in the second line of the coaxial antenna cable, the probe connection then being connected to the impedance transformer to a point where the first and second lines of the coaxial antenna cable are connected together.
